# FASCICULE DE BREVET EUROPEEN

(11) **EP 2 785 260 B1**
(45) Date de publication et mention de la délivrance du brevet: **12.08.2015**
(21) Numéro de dépôt: 12801620.1
(22) Date de dépôt: 09.11.2012
(51) Int. Cl.: A61B 17/16, A61B 17/70

(54) **FORET ARTICULÉ ET SON DISPOSITIF D' ENTRAINEMENT À MOUVEMENT ALTERNATIF**
GELENKBOHRER UND DURCH HUBKOLBEN BEWEGTE ANTRIEBSVORRICHTUNG DAFÜR
ARTICULATED DRILL AND THE RECIPROCATING MOTION DRIVE DEVICE THEREOF

(30) Priorité: 28.11.2011 FR 1160881
(43) Date de publication de la demande: 08.10.2014
(73) Titulaire: Clariance, 62000 Dainville (FR)
(72) Inventeur: TORNIER, Alain, F-38330 Saint Ismier (FR); VIART, Guy, F-62128 Saint Leger (FR); LEROY, Jean Yves, F-62870 Campagne-les-Hesdin (FR); KRIER, Brice, F-62000 Dainville (FR); BILLON, Adrien, F-59790 Ronchin (FR)
(74) Mandataire: Jeannet, Olivier
(86) Numéro de dépôt international: PCT/FR2012/052591
(87) Numéro de publication internationale: WO 2013/079845

(56) Documents cités:
- WO-A1-2010/111246
- US-A1- 2002 183 758
- US-A1- 2011 166 575

## Description

La présente invention est relative à un foret articulé permettant de réaliser des forages de petit rayon de courbure au moyen d'un dispositif d'entrainement à mouvement alternatif.

On connait d'après la demande de brevet FR 11 00199 appartenant au demandeur un foret articulé appartenant au demandeur qui est constitué d'un tube métallique comportant à l'une de ses extrémités un profil articulé formé de boucles concaves et convexes obtenus par l'agencement de dents et de logements régulièrement répartis sur la périphérie dudit tube.

On note que la forme des dents et des logements constituant les boucles ne permet pas au profil articulé de se courber suivant un rayon de faible diamètre.

En effet, lorsque le profil du tube métallique se courbe, le jeu entre les dents à l'intérieur du rayon courbure diminue, tandis que le jeu entre les dents à l'extérieur dudit rayon de courbure augmente.

Dans cette situation, si l'on applique un couple de rotation au tube métallique, il sera transmit uniquement par les dents à l'intérieur du rayon de courbure. Cela aura pour effet de déformer le profil articulé du tube métallique car l'effort n'est pas réparti sur tout le diamètre des différentes sections constituant le profil articulé mais seulement en un seul point. Une fois le profil articulé déformée les dents ne peuvent plus se craboter ensemble car elles ne sont plus positionnées les une en face des autres entrainant la rupture du profil articulé du tube métallique.

WO 2010/111246 concerne des dispositifs destinés à créer une cavité curvilinéaire dans une structure de corps osseux. Le dispositif comprend une extrémité distale combinant perçage et alésage et est constitué d'un foret articulé coopérant avec un dispostif d'entraînement à mouvement oscillatoire.

L'objet de la présente invention consiste à réaliser un foret articulé dont le profil articulé est susceptible de se déformer suivant des rayons de faible courbure sans risque de dé crabotage des dents lorsqu'un couple est appliqué sur le tube métallique.

Le foret articulé suivant la présente invention est particulièrement destiné aux forages de canaux et de canaux à profil courbe ménagés dans des éléments osseux comme par exemple dans la partie osseuse de corps vertébraux d'une colonne vertébrale.

Le foret articulé suivant la présente invention permet de réaliser des canaux à profil courbe dans le corps vertébral des vertèbres afin d'atteindre le disque intervertébral se trouvant entre les vertèbres sus jacente et sous jacente d'un segment rachidien endommagé.

Le dispositif de forage suivant la présente invention est constitué d'un foret articulé pourvue d'un tube métallique comprenant, suivant son axe longitudinale XX', et à l'une de ses extrémités des moyens de fixation coopérant avec un dispositif d'entraînement à mouvement oscittatoire; tandis que l'autre extrémité comporte d'une part un profil comportant une succession de boucles alternativement concaves et convexes assurant la déformation suivant un rayon de courbure **r** dudit profil, et d'autre part au moins une série de dents à bords tranchant.

Le dispositif de forage suivant la présente invention est constitué d'un foret articulé dont la première série de dents présente un bord tranchant qui est incliné par rapport à l'axe longitudinale XX' du tube métallique d'un angle α compris entre 5 et 15 degrés.

Le dispositif de forage suivant la présente invention est constitué d'un foret articulé dont la seconde série de dents présente un bord tranchant qui est incliné par rapport à l'axe longitudinale XX' du tube métallique d'un angle **β** compris entre 55 et 80 degrés.

Le dispositif de forage suivant la présente invention est constitué d'un foret articulé dont le tube métallique comporte dans sa partie interne une gaine de protection en matériau souple déterminant un alésage interne permettant de lisser intérieurement les aspérités et les discontinuités de l'extrémité libre provenant du profil formé par la succession de boucles alternativement concaves et convexes.

Le dispositif de forage suivant la présente invention est constitué d'un foret articulé dont le profil du tube métallique est formé par une combinaison de boucles constituées sur la périphérie dudit tube métallique de dents coopérant respectivement dans des logements de manière à constituer un profil asymétrique et alternativement concaves et convexes.

Le dispositif de forage suivant la présente invention est constitué d'un foret articulé dont le profil du tube métallique comporte sur la fibre ou face extérieure **Fs** du rayon de courbure **r** les dents supérieure, adjacentes, intermédiaires, et latérales et sur la fibre ou face intérieure **Fi** les dents crénelées.

Le dispositif de forage suivant la présente invention est constitué d'un foret articulé dont chaque boucle du profil comporte entre les bords de la dent et les bords du logement correspondant un jeu fonctionnel de déplacement axial positif **Jp,** un jeu fonctionnel de déplacement axial négatif **Jn,** et un jeu fonctionnel d'angulation **Ja** permettant d'assurer audit profil du tube métallique d'une part de se courber progressivement sans dé crabotage de la dent supérieure et des dents adjacentes jusqu'à atteindre le rayon de courbure **r** et d'autre part de résister aux efforts des mouvements oscillatoires.

Le dispositif de forage suivant la présente invention est constitué d'un foret articulé comportant des moyens de fixation qui sont constitués d'un manchon cylindrique coopérant avec un alésage de forme complémentaire et aménagé dans un manchon cylindrique libre en rotation et guidé axialement à l'intérieur d'un boitier support du dispositif d'entrainement.

Le dispositif de forage suivant la présente invention est constitué d'un foret articulé dont le manchon cylindrique des moyens de fixation comporte un méplat pourvue en son milieu d'un cordon d'indexation angulaire coopérant avec un rainure de même profil aménagée dans l'alésage du boitier support.

Le dispositif de forage suivant la présente invention est constitué d'un foret articulé dont le manchon cylindrique des moyens de fixation est pourvu à l'une de ses extrémités d'une butée périphérique venant bloquer l'engagement en translation du tube métallique à l'intérieur de l'alésage.

Le dispositif de forage suivant la présente invention est constitué d'un dispositif d'entraînement dont le boitier support comporte en dessous de l'alésage et à l'opposé de la direction d'introduction du tube métallique des moyens de réception et de fixation d'un moteur électrique.

Le dispositif de forage suivant la présente invention comprend un dispositif d'entrainement comprenant à l'intérieur du boitier support des moyens de transmission permettant de transformer le mouvement de rotation continue du moteur électrique en un mouvement oscillatoire ou alternatif d'amplitude d'au moins 60°degrés.

Le dispositif de forage suivant la présente invention est constitué d'un dispositif d'entrainement dont les moyens de transmission sont constitués d'une première roue dentée solidaire de l'arbre de sortie du moteur électrique qui engrène avec une seconde roue dentée de dimensions plus importante que la première, ladite seconde roue dentée étant montée autour d'un axe de rotation qui est guidé à l'intérieur du boitier support, ladite seconde roue dentée étant solidaire d'un premier vilebrequin coopérant de manière libre en rotation avec la première extrémité d'une tige de liaison dont l'autre extrémité est montée également libre en rotation autour d'un second vilebrequin solidaire du manchon cylindrique de l'alésage pour entrainer ce dernier dans un mouvement oscillatoire.

La description qui va suivre en regard des dessins annexés, donnés à titre d'exemples non limitatifs permettra de mieux comprendre l'invention, les caractéristiques qu'elle présente, et les avantages qu'elle est susceptible de procurer :
Figures 1 et 1a sont des vues illustrant le foret articulé et son dispositif d'entrainement à mouvement alternatif permettant de réaliser des forages de petit rayon de courbure suivant la présente invention.
Figures 2 à 4 sont des vues représentant en détail le profil articulé pourvue d'une extrémité coupante comportant une première et seconde série de dents d'inclinaison différente du foret suivant la présente invention.
Figures 4a et 4b sont des vues illustrant la déformation du profil articulé suivant un rayon de courbure déterminé du foret suivant la présente invention.

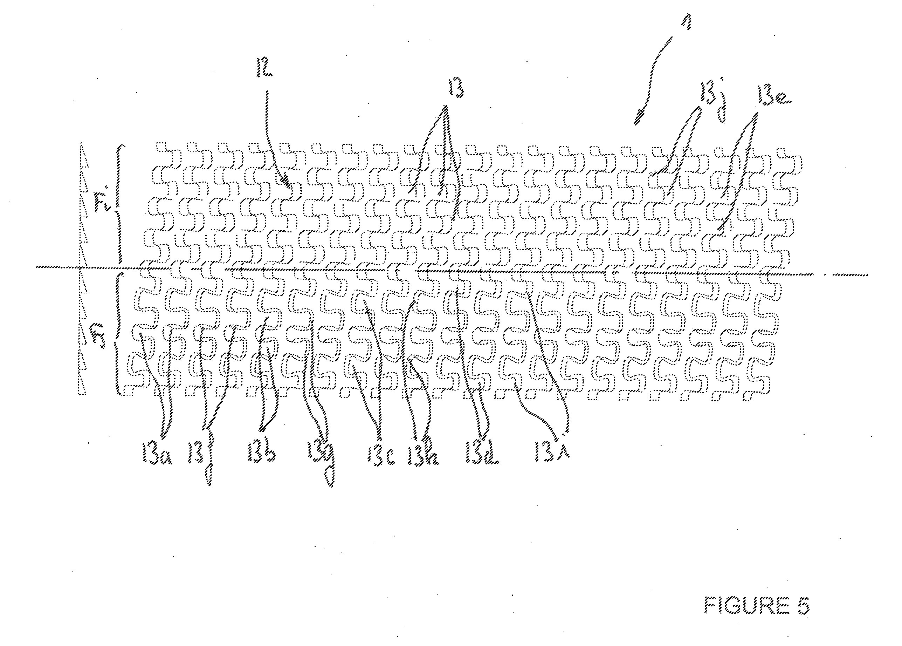

Figure 5 est une vue dépliée montrant la forme de chacune des boucles alternativement concaves et convexes du profil articulé du foret suivant la présente invention.
Figures 6 et 7 sont des vues illustrant en détail la forme des boucles alternativement concaves et convexes des dents supérieures du profil articulé du foret suivant la présente invention.
Figures 8 et 9 sont de vues représentant en détail la forme des boucles alternativement concaves et convexes des dents adjacentes du profil articulé du foret suivant la présente invention.
Figures 10 et 11 sont de vues montrant en détail la forme des boucles alternativement concaves et convexes des dents intermédiaires du profil articulé du foret suivant la présente invention.
Figures 12 et 13 sont de vues montrant en détail la forme des boucles alternativement concaves et convexes des dents latérales du profil articulé du foret suivant la présente invention.
Figures 14 et 15 sont de vues illustrant en détail la forme des boucles alternativement concaves et convexes des dents crénelées du profil articulé du foret suivant la présente invention.
Figure 16 est une vue en perspective éclaté représentant les moyens d'accouplement du foret articulé dans son dispositif d'entrainement à mouvement alternatif suivant la présente invention.
Figure 17 est une vue en perspective montrant les moyens d'entrainement du dispositif d'entrainement à mouvement alternatif suivant la présente invention.

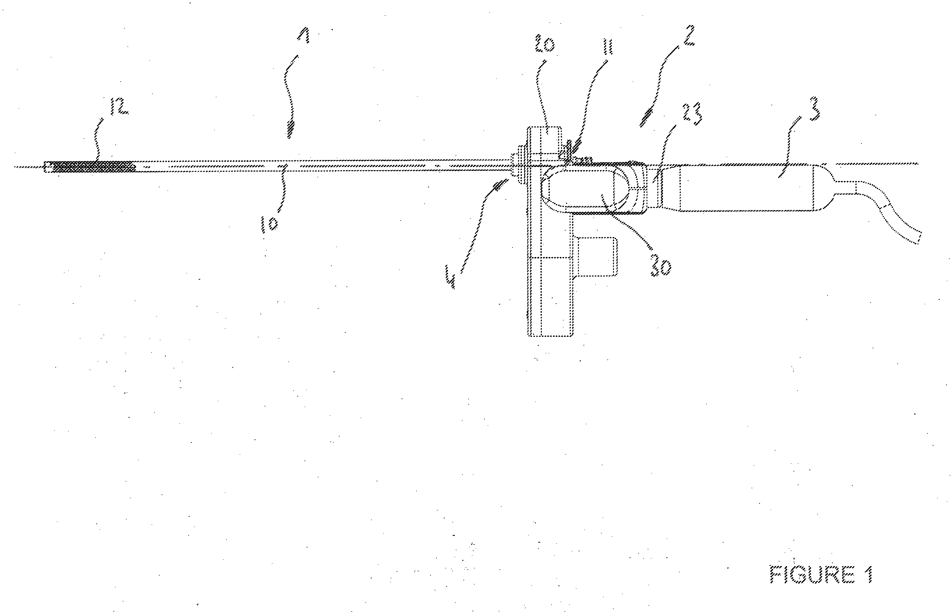

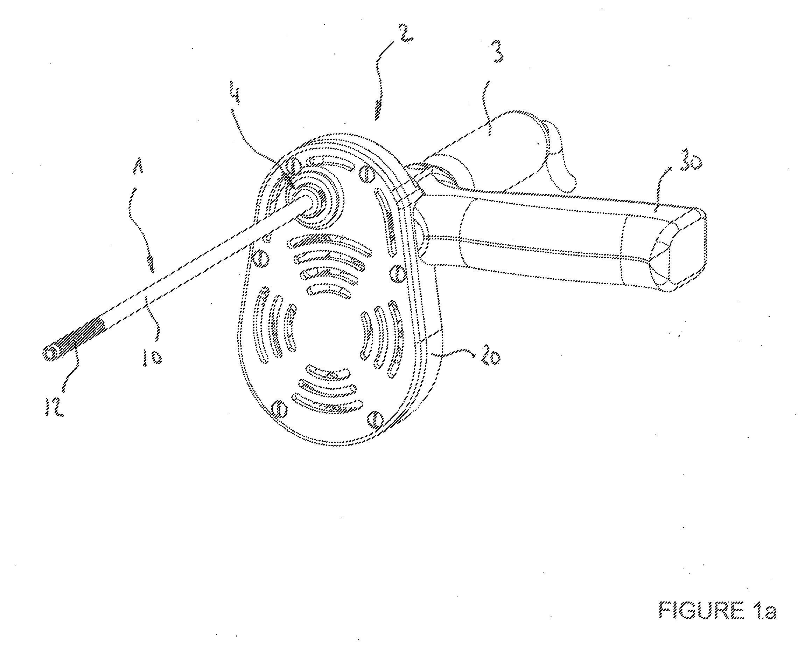

On a montré en figures 1 et 1a un foret articulé 1 agencé sur un dispositif d'entrainement à mouvement alternatif 2 assurant les mouvements oscillatoires dudit foret autour de son axe longitudinal XX'.

Le foret articulé 1 est constitué d'un tube cylindrique et métallique 10 présentant à l'une de ses extrémités des moyens de fixation 11 coopérant avec un profil de forme complémentaire aménagé dans le dispositif d'entrainement 2, tandis que l'autre extrémité est découpée suivant un profil 12 qui peut être constitué par exemple d'une succession de boucles 13 alternativement concaves et convexes assurant la déformation et l'articulation suivant une forme courbe de rayon **r** de ladite extrémité.

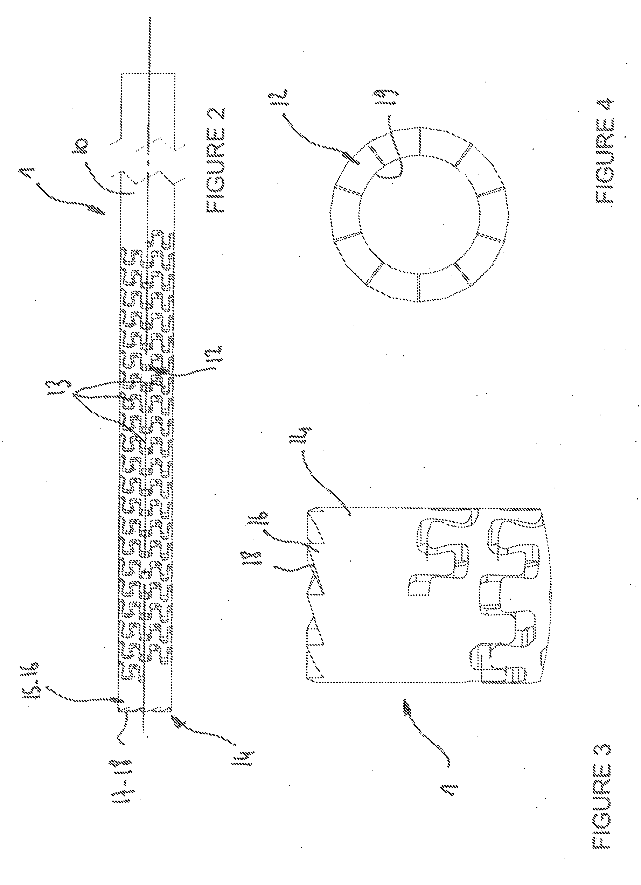

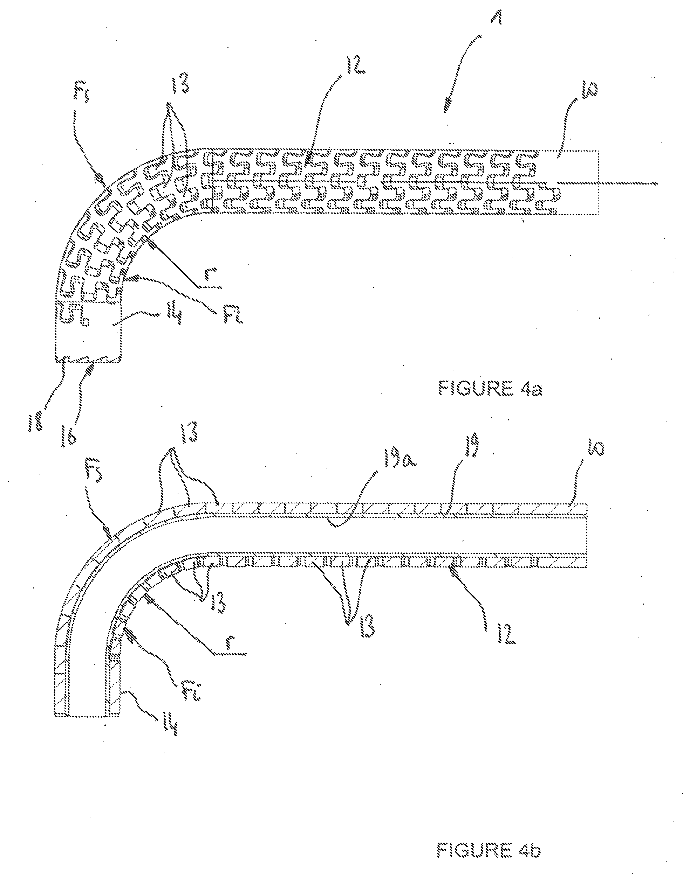

On a représenté en figures 2 à 4, 4a, 4b le tube métallique 10 se terminant après le profil 12 par une extrémité coupante 14 qui comporte par exemple, une première et seconde série de dents 15, 16 présentant respectivement des bords tranchant 17, 18 d'inclinaison différente.

La première série de dents 15 présente un bord tranchant 17 qui est incliné par rapport à l'axe longitudinale XX' du tube métallique 10 d'un angle **α** compris entre 5 et 15 degrés.

La seconde série de dents 16 présente un bord tranchant 18 qui est incliné par rapport à l'axe longitudinale XX' du tube métallique 10 d'un angle **β** compris entre 55 et 80 degrés.

A titre d'exemple, non limitatif l'extrémité coupante 14 peut comprendre au moins une série de dent à bords tranchants.

On note également que le pas de la dent est suffisamment petit pour permettre lors du mouvement alternatif que le déplacement d'une dent chevauche au moins la découpe de la dent précédente.

Le foret articulé 1 comporte dans la partie interne du tube métallique 10 une gaine de protection 19 en matériau souple déterminant un alésage interne 19**a** permettant de lisser intérieurement les aspérités et les discontinuités de l'extrémité libre provenant du profil 12 formé, par exemple par la succession de boucles 13 alternativement concaves et convexes (figures 4 et 4b).

La gaine de protection 19 peut être réalisée, par exemple, dans un matériau souple tel que du PTFE assurant également un coefficient de glissement important permettant le coulissement d'élément de guidage ou analogue (figures 4 et 4b).

On a illustré en figures 4**a** et 5 le profil 12 du foret articulé 1 qui est formé, par exemple par une combinaison de boucles 13 constituées sur la périphérie du tube métallique 10 de dents 13**a** à 13**e** coopérant respectivement dans des logements 13**f** à 13**i** de manière à constituer un profil asymétrique et alternativement concaves et convexes.

Le profil 12 comporte en fonction du rayon de courbure **r** à obtenir du foret articulé 1 d'une part sur la périphérie du tube métallique 10 un nombre de boucle 13 qui est compris entre six et huit et d'autre part entre l'extrémité coupante 14 et le début dudit profil 12 un nombre de révolution compris entre quinze et vingt cinq.

Selon un mode préféré de l'invention, le foret articulé 1 comporte un profil 12 qui est constitué sur la périphérie du tube métallique 10 d'au moins sept boucles 13 disposée sur la fibre ou face extérieure **Fs** et d'au moins sept autres boucles 13 sur la fibre ou face intérieure **Fi** lorsque ledit profil 12 est déformé par rapport à l'axe XX' suivant le rayon de courbure **r** (figure 4a).

Egalement selon un mode préféré de l'invention, le foret articulé comporte un profil 12 comprenant entre l'extrémité coupante 14 et le début dudit profil 12 un nombre de vingt et une révolution de boucles 13 asymétrique.

La découpe asymétrique des boucles 13 permet d'adapter la forme de chaque dent 13**a** à 13**e** en fonction de sa position sur le tube métallique 10. En effet la longueur de la dent supérieure 13**a** est adaptée afin que le couple soit transmis par toutes les dents 13**b** à 13**e** se trouvant sur la périphérie du tube métallique 10.

Egalement la combinaison de la longueur de la dent supérieur 13**a**, du diamètre du tube métallique 10 et des jeux fonctionnels J**a**, J**p**, **Jn** de chaque dent permettent de définir le rayon de courbure maximum **r** du profil 12.

Ainsi, il est prévu lorsque le profil 12 du tube métallique 10 est en position droite et non déformé un très faible jeu fonctionnel des dents 13**a** à 13**d** se trouvant sur la fibre ou face extérieure **Fs** et un jeu fonctionnel plus important sur les dents 13**e** disposée sur la fibre ou face intérieure **Fi.** A l'inverse lorsque le profil 12 du tube métallique 10 est déformé suivant le rayon de courbure **r**, il est prévu du fait de la forme des dents 13**a** à 13**e** un jeu fonctionnel important au niveau de la fibre ou face extérieure **Fs** et un faible jeu fonctionnel au niveau de la fibre intérieure **Fi.**

Le profil 12 du foret articulé 1 comporte, par exemple, sur la fibre ou face extérieure **Fs** sept boucles asymétriques 13 constituant :
- Une dent supérieure 13**a**,
- Deux dents adjacentes 13**b**,
- Deux dents intermédiaires 13**c**,
- Deux dents latérales 13**d**.

Egalement le profil 12 du foret articulé 1 comporte, par exemple, sur la fibre ou face intérieure **Fi** sept boucles asymétriques 13 constituants :
- Sept dents crénelées 13**e**.

Du fait de cet agencement particulier des boucles asymétriques 13 sur la périphérie du tube métallique 10 et plus particulièrement au niveau des fibres ou face extérieure **Fs** et intérieure **Fi,** le foret articulé 1 peut être entrainé par le dispositif d'entraînement 2 dans un mouvement de rotation alternatif qui peut être de plus ou moins 60° degrés soit une amplitude de 120° degrés.

Le profil de chaque dent 13**a** à 13**e** présente un jeu fonctionnel croissant entre l'axe longitudinale du tube cylindrique 10 et la fibre ou face interne **Fi** du rayon de courbure **r** favorisant l'engrènement des boucles 13 entre elles lors d'une déformation du profil 12 et plus particulièrement lors d'une déformation suivant un rayon de courbure **r** compris entre cinq et quinze millimètres.

En effet il impératif lors de cette déformation du profil 12 et de l'entrainement selon un mouvement de rotation alternatif du tube métallique 10 que chaque dent 13**a** à 13**e** soit toujours en contact entres elles sans risque de désunion ou de dé-crabotage de ces dernières.

Pour cela chaque boucle 13 comporte entre les bords de la dent 13**a** à 13**e** et les bords du logement 13**f** à 13**i** correspondant un jeu fonctionnel de déplacement axial positif **Jp,** un jeu fonctionnel de déplacement axial négatif **Jn**, et un jeu fonctionnel d'angulation **Ja.**

Les jeux fonctionnels de déplacement axial positif **Jp** et négatif **Jn** et le jeu fonctionnel d'angulation **Ja** permettent d'assurer au profil 12 du tube métallique 10 d'une part de se courber progressivement jusqu'à atteindre le rayon de courbure **r** de fonctionnement et de perçage et d'autre part de résister aux efforts des mouvements alternatifs.

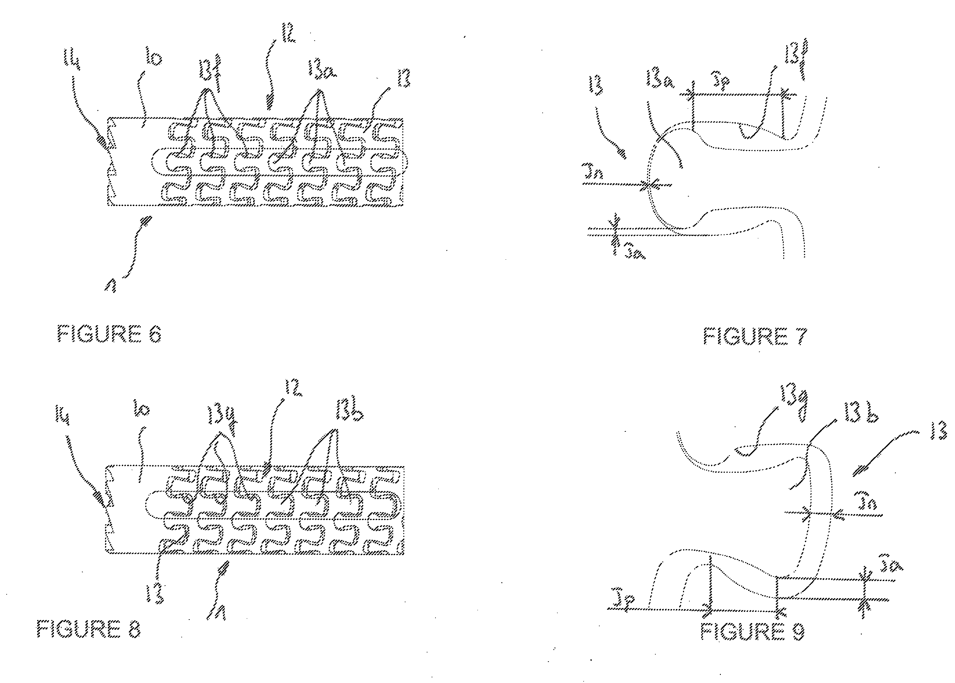

On a montré en figures 6 et 7 une série de boucles 13 constituant sur la longueur du profil 12 des dents 13**a** dites « dents supérieures » à profil concave coopérant dans des logements 13**f** de forme complémentaire convexe.

Chaque dent supérieure 13**a** présente un profil concave dirigé en direction de l'extrémité coupante 14 du tube métallique 10, tandis que le logement 13**f** est dirigé dans une direction opposée.

Sur l'ensemble des dents 13**a** à 13**e** constituant le profil 12, la dent supérieure 13a est celle qui a le plus grand jeu de déplacement axial positif **Jp** d'environ 75/100 à 80/100 de millimètres du fait de sa position sur la périphérie du tube métallique 10. En effet les dents supérieures 13**a** se trouvent toutes sur la fibre ou face extérieure **Fs** du rayon de courbure **r**.

A cet effet, plus le profil 12 du tube métallique 10 se courbe, plus le jeu de déplacement axial positif **Jp** diminue jusqu'à atteindre la courbure maximale dudit rayon de courbure **r** et un jeu de déplacement axial positif **Jp** proche de zéro.

Par contre, chaque dent supérieure 13**a** présente avec son logement 13**f** un jeu fonctionnel de déplacement axial négatif **Jn** qui est d'environ 2/100 à 4/100 de millimètres car cette dernière du fait de sa position ne travail jamais en compression. Ce jeu fonctionnel de déplacement axial négatif **Jn** est le plus faible par rapport à ceux appliqués aux autres dents.

Le jeu fonctionnel d'angulation **Ja** est d'environ 5/100 à 8/100 de millimètres, car chaque dent supérieure 13**a** n'a pas besoin de pivoter dans son logement 13**f**. En effet, en position extrême du mouvement rotatif alternatif, chaque dent supérieure 13**a** ne se trouve jamais dans une position défavorable du fait de son positionnement axial lors de la déformation du profil 12 du tube métallique 10. Chaque dent supérieure 13**a** a pour fonction de transmettre le couple issu du mouvement rotatif alternatif du dispositif d'entraînement 2 assurant au foret articulé 1 le perçage du canal à profil courbe.

Le profil de chaque dent supérieure 13**a** et de chaque logement 13**f** permettent d'éviter lors de la déformation du profil 12 et de l'entraînement en rotation alternative du tube métallique 10 le dé-crabotage desdites dents et le risque de rupture du foret articulé 1

On a représenté en figures 8 et 9 une série de boucles 13 constituant sur la longueur du profil 12 des dents 13**b** dites « dents adjacentes » à profil concave coopérant dans des logements 13**g** de forme complémentaire convexe.

Les dents adjacentes 13**b** et les logements 13**g** se situe dans le prolongement et de part et d'autre des dents supérieures 13**a** du profil 12.

Les dents adjacentes 13**b** et les logements 13**g** sont dirigés respectivement dans une direction opposée à celle des dents supérieures13**a** et des logements 13**f**.

Chaque dent adjacente 13**b** comporte avec son logement 13**g** un jeu de déplacement axial positif **Jp** semblable à celui des dents supérieures 13**a** d'environ 75/100 à 80/100 de millimètres du fait de sa position sur la périphérie du tube métallique 10. En effet les dents adjacentes 13**b** se trouvent toutes sur la fibre ou face extérieure **Fs** du rayon de courbure **r**.

Chaque dent adjacente 13**b** comporte avec son logement 13**g** un jeu fonctionnel de déplacement axial négatif **Jn** qui est d'environ 15/100 à 25/100 de millimètres pour assurer un travail en compression de chacune des dites dents adjacentes13**b**.

Chaque dent adjacente 13**b** comporte avec son logement 13**g** un jeu d'angulation **Ja** qui est compris entre 10/100 et 12/100 de millimètres permettant d'assurer la rotation de la dent dans son logement lorsqu'elle atteint en position extrême une angulation importante.

Chaque dent adjacentes 13**b** a pour fonction de transmettre le couple issu du mouvement rotatif alternatif du dispositif d'entraînement 2 assurant au foret articulé 1 le perçage du canal à profil courbe.

Le profil de chaque dent adjacentes 13**b** et de chaque logements 13**g** permettent d'éviter lors de la déformation du profil 12 et de l'entraînement en rotation alternative du tube métallique 10 le dé-crabotage desdites dents et le risque de rupture du foret articulé 1

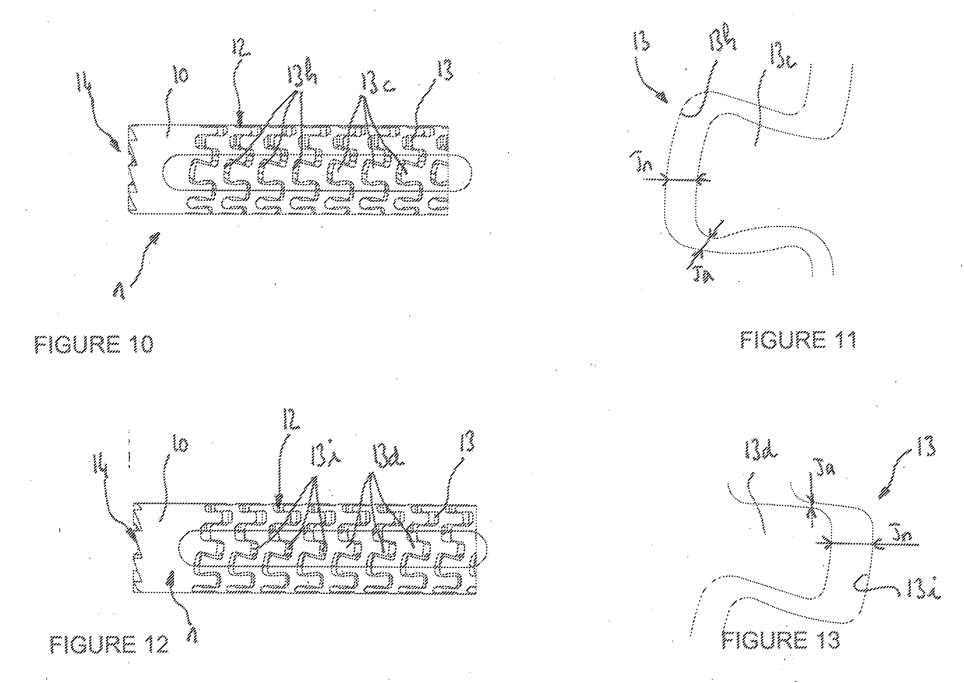

On a illustré en figures 10 et 11 une série de boucles 13 constituant sur la longueur du profil 12 des dents 13**c** dites « dents intermédiaires » à profil concave coopérant dans des logements 13**h** de forme complémentaire convexe. Les dents intermédiaires 13**c** et les logements 13**h** se situe dans le prolongement et de part et d'autre des dents adjacentes 13**b** et des logements 13**g** correspondants du profil 12.

Les dents intermédiaires 13**c** et les logements 13**h** sont dirigés respectivement dans la même direction que celle des dents supérieures13**a** et des logements 13**f**, c'est-à-dire en direction de l'extrémité coupante 14 en ce qui concerne lesdites dents intermédiaires 13**c**.

Chaque dent intermédiaire 13**c** comporte avec son logement 13**h** un jeu de déplacement axial positif **Jp** infini lui permettant de se déloger du fait de sa position sur la périphérie du tube métallique 10. En effet les dents intermédiaires 13**c** se trouvent toutes sur la fibre ou face extérieure **Fs** du rayon de courbure **r**.

Chaque dent intermédiaire 13**c** comporte avec son logement 13**h** un jeu fonctionnel de déplacement axial négatif **Jn** qui est d'environ 15/100 à 35/100 de millimètres pour assurer un travail en compression de chacune des dites dents.

Chaque dent intermédiaire 13**c** comporte avec son logement 13**h** un jeu d'angulation **Ja** qui est compris entre 10/100 et 15/100 de millimètres permettant d'assurer l'inclination de la dent dans son logement lorsqu'elle atteint en position extrême une angulation importante.

Chaque dent intermédiaire 13**c** a pour fonction de transmettre le couple issu du mouvement rotatif alternatif du dispositif d'entraînement 2 assurant au foret articulé 1 le perçage du canal à profil courbe.

Egalement chaque dent intermédiaire 13**c** du fait de son profil détermine l'amplitude du tube métallique 10 et du profil 12 et assure la sécurité des autres dents en position extrême car elle sert de butée.

On a montré en figures 12 et 13 une série de boucles 13 constituant sur la longueur du profil 12 des dents 13**d** dites « dents latérales » à profil concave coopérant dans des logements 13**i** de forme complémentaire convexe.

Les dents latérales 13**d** et les logements 13**i** se situent dans le prolongement,et de part et d'autre des dents intermédiaires 13**c** et des logements 13**h** correspondants du profil 12.

Chaque dent latérale 13**d** comporte avec son logement 13**i** un jeu de déplacement axial positif **Jp** infini lui permettant de se déloger du fait de la faible longueur de ladite dent et de sa position sur la périphérie du tube métallique 10.

En effet les dents latérales 13**d** se trouvent toutes sur la fibre ou face extérieure **Fs** du rayon de courbure **r**.

Chaque dent latérale 13**d** comporte avec son logement 13**i** un jeu fonctionnel de déplacement axial négatif **Jn** qui est d'environ 25/100 à 45/100 de millimètres assurant la courbure souhaitée du profil 12 en position extrême.

Chaque dent latérale 13**d** comporte avec son logement 13**i** un faible jeu d'angulation **Ja** qui est compris entre 2/100 et 8/100 de millimètres permettant d'assurer en combinaison avec sa faible longueur l'inclination et le pivotement de ladite dent dans son logement.

Chaque dent latérale 13**d** a pour fonction de transmettre le couple issu du mouvement rotatif alternatif du dispositif d'entraînement 2 assurant au foret articulé 1 le perçage du canal à profil courbe.

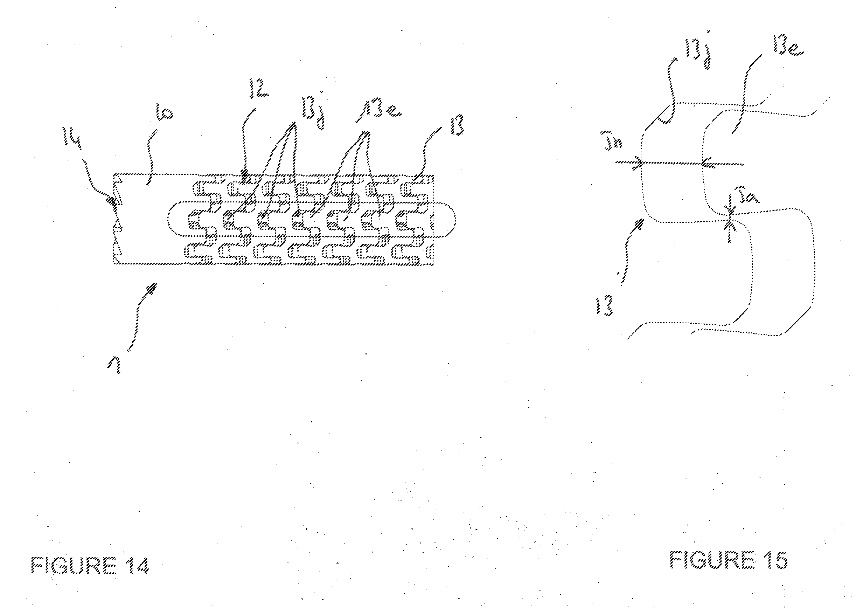

On a représenté en figures 14 et 15 une série de boucles 13 constituant sur la longueur du profil 12 des dents 13**e** dites « dents crénelées» à profil concave coopérant dans des logements 13**j** de forme complémentaire convexe.

Les dents crénelées 13**e** et les logements 13**j** se situent dans le prolongement et de part et d'autre des dents latérales 13**d** et des logements 13**i** correspondants du profil 12 de manière à se trouver sur la face ou fibre interne **Fi** du rayon de courbure r du profil 12.

Chaque dent crénelée 13**e** comporte avec son logement 13**i** un jeu de déplacement axial positif **Jp** infini lui permettant de se déloger du fait de la faible longueur de ladite dent et de sa position sur la périphérie du tube métallique 10.

Chaque dent crénelée 13**e** comporte avec son logement 13**i** un jeu fonctionnel de déplacement axial négatif **Jn** qui est d'environ 70/100 à 80/100 de millimètres assurant la courbure souhaitée du profil 12 en position extrême.

La longueur de chaque dent crénelée 13**e** est déterminée de manière que cette dernière présente le plus grand jeu fonctionnel de déplacement axial négatif **Jn** tout en gardant l'engrènement desdites dents quand le profil 12 du tube métallique 10 est en position droite, c'est à dire non courbé.

Chaque dent crénelée 13**e** comporte avec son logement 13**j** un faible jeu d'angulation **Ja** qui est compris entre 2/100 et 8/100 de millimètres permettant d'assurer en combinaison avec sa faible longueur l'inclination et le pivotement de ladite dent dans son logement.

Le faible jeu d'angulation **Ja** de chaque dent crénelée 13**e** permet d'éviter la perte d'amplitude, c'est-à-dire qu'elle participe au rattrapage des jeux fonctionnels durant la motorisation du foret articulé 1 dans un mouvement rotatif alternatif.

Chaque dent crénelée 13**e** a pour fonction de transmettre le couple issu du mouvement rotatif alternatif du dispositif d'entraînement 2 assurant au foret articulé 1 le perçage du canal à profil courbe.

On note que le profil particulier de chacune des dents supérieures 13**a** adjacentes 13**b**, intermédiaires 13**c**, latérales 13**d** et crénelés 13**e** du profil 12 permet de transmettre l'intégralité du couple en position droite ou courbé du foret articulé 1 lors du perçage du canal.

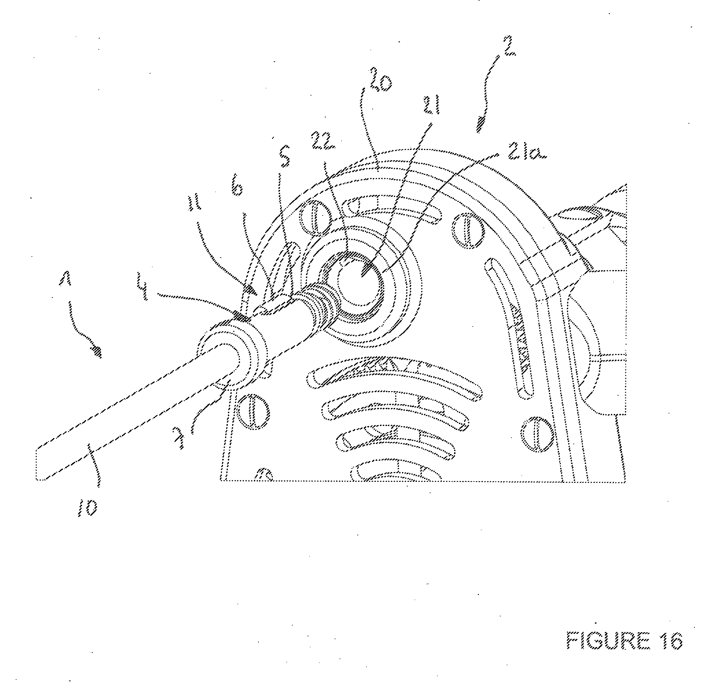

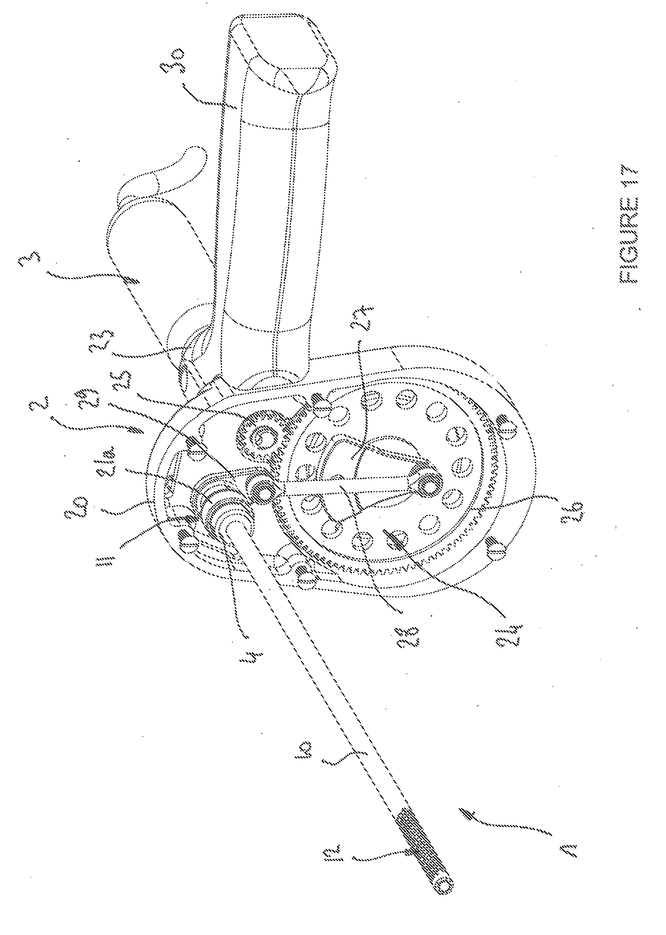

On a illustré en figures 1, 1a, 16 et 17 le dispositif d'entraînement à mouvement alternatif 2 assurant les mouvements oscillatoires du foret articulé 1 autour de son axe longitudinal XX'.

Le foret articulé 1 est agencé sur le dispositif d'entrainement 2 toujours dans la même position axiale de manière à positionner le profil 12 toujours dans la même position de courbure afin que les dents 13**a** à 13**d** se trouvent sur la fibre ou face extérieure **Fs** du rayon de courbure **r** et les dents crénelées 13**e** soient disposées sur la fibre ou face intérieur **Fi** dudit rayon de courbure **r**.

Pour cela le foret articulé 1 comporte à l'opposé de l'extrémité coupante 14 une extrémité solidaire de moyen de fixation 11 coopérant avec un alésage 21 de forme complémentaire et aménagé dans un manchon cylindrique 21**a** libre en rotation et guidé axialement à l'intérieur du boitier support 20 du dispositif d'entrainement 2.

Les moyens de fixation 11 sont constitué d'un manchon cylindrique 4 comportant sur face externe un méplat 5 pourvue en son milieu d'un cordon d'indexation angulaire 6 coopérant avec un rainure 22 de même profil aménagée dans l'alésage 21 du boitier support 20. Le manchon cylindrique 4 est pourvue à l'une de ses extrémité une butée périphérique 7 venant bloquer l'engagement en translation du foret articulé 1 à l'intérieur de l'alésage 21.

Le boitier support 20 du dispositif d'entrainement 2 comporte en dessous de l'alésage 21 et à l'opposé de la direction d'introduction du foret articulé 1 des moyens de réception et de fixation 23 d'un moteur électrique 3.

Le dispositif d'entrainement 2 comporte à l'intérieur du boitier support 20 des moyens de transmission 24 permettant de transformer le mouvement de rotation continue du moteur électrique 3 en un mouvement oscillatoire ou alternatif d'amplitude d'environs 120°degrés.

Les moyens de transmission 24 sont constitués d'une première roue dentée 25 solidaire de l'arbre de sortie du moteur électrique 3 qui engrène avec une seconde roue dentée 26 de dimensions plus importante que la première.

La seconde roue dentée 26 est montée autour d'un axe de rotation qui est guidé à l'intérieur du boitier support 20.

La seconde roue dentée 26 est solidaire d'un premier vilebrequin 27 coopérant de manière libre en rotation avec la première extrémité d'une tige de liaison 28 dont l'autre extrémité est montée également libre en rotation autour d'un second vilebrequin 29 solidaire du manchon cylindrique 21**a** de l'alésage 21 pour entrainer ce dernier dans un mouvement oscillatoire.

Le fonctionnement du dispositif d'entraînement 2 se comprend aisément de la description qui précède à savoir que ce dernier permet lorsque le foret articulé 1 est engagé dans l'alésage 21 du boitier support 20 d'être entrainé dans un mouvement oscillatoire d'amplitude d'environs 120°degrés.

Le dispositif d'entrainement 2 comporte au niveau des moyens de réception 23 et suivant une direction perpendiculaire à ces derniers une poignée de préhension 30 permettant la manipulation dudit dispositif solidaire du foret articulé 1.

Il doit d'ailleurs être entendu que la description qui précède n'a été donnée qu'a titre d'exemple et qu'elle ne limite nullement le domaine de l'invention dont on ne sortirait pas en remplaçant les détails d'exécution décrits par tous autres équivalents.

## Revendications

1. Dispositif de forage pour l'aménagement d'un canal à profil courbe à l'intérieur du corps d'une vertèbre d'un segment rachidien d'une colonne vertébrale à instrumenter, **caractérisé en ce qu'**il est constitué d'un foret articulé (1) pourvue d'un tube métallique (10) comprenant, suivant son axe longitudinale XX', et à l'une de ses extrémités des moyens de fixation (11) coopérant avec un dispositif d'entraînement à mouvement oscillatoire (2), tandis que l'autre extrémité comporte d'une part un profil (12) comportant une succession de boucles (13) alternativement concaves et convexes assurant la déformation suivant un rayon de courbure **r** dudit profil, et d'autre part au moins une série de dents (15, 16) à bords tranchant (17, 18).

2. Dispositif de forage suivant la revendication 1, **caractérisé en ce que** la première série de dents (15) présente un bord tranchant (17) qui est incliné par rapport à l'axe longitudinale XX' du tube métallique (10) d'un angle **α** compris entre 5 et 15 degrés.

3. Dispositif de forage suivant la revendication 2, **caractérisé en ce que** la seconde série de dents (16) présente un bord tranchant (18) qui est incliné par rapport à l'axe longitudinale XX' du tube métallique (10) d'un angle **β** compris entre 55 et 80 degrés.

4. Dispositif de forage suivant la revendication 1, **caractérisé en ce que** le tube métallique (10) comporte dans sa partie interne une gaine de protection (19) en matériau souple déterminant un alésage interne (19**a**) permettant de lisser intérieurement les aspérités et les discontinuités de l'extrémité libre provenant du profil (12) formé par la succession de boucles (13) alternativement concaves et convexes

5. Dispositif de forage suivant la revendication 1, **caractérisé en ce que** le profil (12) est formé par une combinaison de boucles (13) constituées sur la périphérie du tube métallique (10) de dents (13**a** à 13**e**) coopérant respectivement dans des logements (13**f** à 13**j**) de manière à constituer un profil asymétrique et alternativement concaves et convexes.

6. Dispositif de forage suivant la revendication 5, **caractérisé en ce que** le profil (12) comporte sur la fibre ou face extérieure **Fs** du rayon de courbure **r** les dents supérieure (13**a**), adjacentes (13**b**), intermédiaires (13**c**), et latérales (13**d**) et sur la fibre ou face intérieure **Fi** les dents crénelées (13**e**).

7. Dispositif de forage suivant la revendication 5, **caractérisé en ce que** chaque boucle (13) comporte entre les bords de la dent (13**a à** 13**e**) et les bords du logement (13**f** à 13**j**) correspondant un jeu fonctionnel de déplacement axial positif **Jp,** un jeu fonctionnel de déplacement axial négatif **Jn,** et un jeu fonctionnel d'angulation **Ja** permettant d'assurer au profil (12) du tube métallique (10) d'une part de se courber progressivement sans dé crabotage de la dent supérieure (13**a**) et des dents adjacentes (13**b**) jusqu'à atteindre le rayon de courbure **r** et d'autre part de résister aux efforts des mouvements oscillatoires.

8. Dispositif de forage suivant la revendication 1, **caractérisé en ce que** les moyens de fixation (11) sont constitués d'un manchon cylindrique (4) coopérant avec un alésage (21) de forme complémentaire et aménagé dans un manchon cylindrique (21**a**) libre en rotation et guidé axialement à l'intérieur d'un boitier support (20) du dispositif d'entrainement (2).

9. Dispositif de forage suivant la revendication 8, **caractérisé en ce que** le manchon cylindrique (4) comporte un méplat (5) pourvu en son milieu d'un cordon d'indexation angulaire (6) coopérant avec un rainure (22) de même profil aménagée dans l'alésage (21) du boitier support (20).

10. Dispositif de forage suivant la revendication 8, **caractérisé en ce que** le manchon cylindrique (4) est pourvu à l'une de ses extrémité d'une butée périphérique (7) venant bloquer l'engagement en translation du tube métallique (10) à l'intérieur de l'alésage (21).

11. Dispositif de forage suivant la revendication 8, **caractérisé en ce que** le boitier support (20) du dispositif d'entrainement (2) comporte en dessous de l'alésage (21) et à l'opposé de la direction d'introduction du tube métallique (10) des moyens de réception et de fixation (23) d'un moteur électrique (3).

12. Dispositif de forage suivant la revendication 8, **caractérisé en ce que** le dispositif d'entrainement (2) comporte à l'intérieur du boitier support (20) des moyens de transmission (24) permettant de transformer le mouvement de rotation continue du moteur électrique (3) en un mouvement oscillatoire ou alternatif d'amplitude d'au moins 60°degrés.

13. Dispositif de forage suivant la revendication 8, **caractérisé en ce que** les moyens de transmission (24) sont constitués d'une première roue dentée (25) solidaire de l'arbre de sortie du moteur électrique (3) qui engrène avec une seconde roue dentée (26) de dimensions plus importante que la première, ladite seconde roue dentée (26) étant montée autour d'un axe de rotation qui est guidé à l'intérieur du boitier support (20), ladite seconde roue dentée 26 étant solidaire d'un premier vilebrequin (27) coopérant de manière libre en rotation avec la première extrémité d'une tige de liaison (28) dont l'autre extrémité est montée également libre en rotation autour d'un second vilebrequin (29) solidaire du manchon cylindrique (21**a**) de l'alésage (21) pour entrainer ce dernier dans un mouvement oscillatoire.

## Patentansprüche

1. Bohrvorrichtung zur Einrichtung eines Kanals mit gekrümmtem Profil im Inneren des Körpers eines Wirbels eines Spinalsegments einer zu instrumentierenden Wirbelsäule, **dadurch gekennzeichnet, dass** sie aus einem Gelenkbohrer (1) besteht, der mit einem Metallrohr (10) versehen ist, das entlang seiner Längsachse XX' und an einem seiner Enden Befestigungseinrichtungen (11) enthält, die mit einer Antriebsvorrichtung mit Schwingbewegung (2) zusammenwirken, während das andere Ende einerseits ein Profil (12) mit einer Folge von abwechselnd konkaven und konvexen Schleifen (13), die die Verformung des Profils gemäß einem Krümmungsradius **r** gewährleisten, und andererseits mindestens eine Reihe von Zähnen (15, 16) mit Schneidkanten (17, 18) aufweist.

2. Bohrvorrichtung nach Anspruch 1, **dadurch gekennzeichnet, dass** die erste Reihe von Zähnen (15) eine Schneidkante (17) aufweist, die bezüglich der Längsachse XX' des Metallrohrs (10) um einen Winkel α zwischen 5 und 15 Grad geneigt ist.

3. Bohrvorrichtung nach Anspruch 2, **dadurch gekennzeichnet, dass** die zweite Reihe von Zähnen (16) eine Schneidkante (18) aufweist, die bezüglich der Längsachse XX' des Metallrohrs (10) um einen Winkel β zwischen 55 und 80 Grad geneigt ist.

4. Bohrvorrichtung nach Anspruch 1, **dadurch gekennzeichnet, dass** das Metallrohr (10) in seinem Innenbereich eine Schutzhülle (19) aus geschmeidigem Material aufweist, die eine innere Bohrung (19a) bestimmt, welche es ermöglicht, die Rauigkeiten und Unstetigkeiten des freien Endes innen zu glätten, die von dem von der Folge von abwechselnd konkaven und konvexen Schleifen (13) gebildeten Profil (12) stammen.

5. Bohrvorrichtung nach Anspruch 1, **dadurch gekennzeichnet, dass** das Profil (12) von einer Kombination von Schleifen (13) gebildet wird, die auf dem Umfang des Metallrohrs (10) aus Zähnen (13a bis 13e) bestehen, die je in Aufnahmen (13f bis 13i) so zusammenwirken, dass sie ein unsymmetrisches Profil bilden und abwechselnd konkav und konvex sind.

6. Bohrvorrichtung nach Anspruch 5, **dadurch gekennzeichnet, dass** das Profil (12) auf der äußeren Faser oder Seite Fs des Krümmungsradius r die oberen (13a), benachbarten (13b), zwischenliegenden (13c) und seitlichen Zähne (13d) und auf der inneren Faser oder Seite Fi die gezackten Zähne (13e) aufweist.

7. Bohrvorrichtung nach Anspruch 5, **dadurch gekennzeichnet, dass** jede Schleife (13) zwischen den Rändern des Zahns (13a bis 13e) und den Rändern der entsprechenden Aufnahme (13f bis 13j) ein funktionelles Spiel positiver axialer Verschiebung Jp, ein funktionelles Spiel negativer axialer Verschiebung Jn und ein funktionelles Winkelungsspiel Ja aufweist, das es ermöglicht, dem Profil (12) des Metallrohrs (10) einerseits zu gewährleisten, dass es sich progressiv ohne Auskupplung des oberen Zahns (13a) und der benachbarten Zähne (13b) krümmt, bis es den Krümmungsradius r erreicht, und andererseits zu gewährleisten, dass es den Kräften der Schwingbewegungen widersteht.

8. Bohrvorrichtung nach Anspruch 1, **dadurch gekennzeichnet, dass** die Befestigungseinrichtungen (11) aus einer zylindrischen Muffe (4) bestehen, die mit einer Bohrung (21) zusammenwirkt, die eine komplementäre Form hat und in einer zylindrischen Muffe (21a) ausgespart ist, die drehfrei ist und axial im Inneren eines Trägergehäuses (20) der Antriebsvorrichtung (2) geführt wird.

9. Bohrvorrichtung nach Anspruch 8, **dadurch gekennzeichnet, dass** die zylindrische Muffe (4) eine Abflachung (5) aufweist, die in ihrer Mitte mit einem Winkelindexierungswulst (6) versehen ist, der mit einer Rille (22) gleichen Profils zusammenwirkt, die in der Bohrung (21) des Trägergehäuses (20) ausgespart ist.

10. Bohrvorrichtung nach Anspruch 8, **dadurch gekennzeichnet, dass** die zylindrische Muffe (4) an einem ihrer Enden mit einem Umfangsanschlag (7) versehen ist, der das Einführen in Translationsrichtung des Metallrohrs (10) ins Innere der Bohrung (21) blockiert.

11. Bohrvorrichtung nach Anspruch 8, **dadurch gekennzeichnet, dass** das Trägergehäuse (20) der Antriebsvorrichtung (2) unter der Bohrung (21) und entgegengesetzt zur Einführrichtung des Metallrohrs (10) Aufnahme- und Befestigungseinrichtungen (23) eines Elektromotors (3) aufweist.

12. Bohrvorrichtung nach Anspruch 8, **dadurch gekennzeichnet, dass** die Antriebsvorrichtung (2) im Inneren des Trägergehäuses (20) Übertragungseinrichtungen (24) aufweist, die es ermöglichen, die kontinuierliche Drehbewegung des Elektromotor (3) in eine Schwingoder alternative Bewegung einer Amplitude von mindestens 60 Grad umzuwandeln.

13. Bohrvorrichtung nach Anspruch 8, **dadurch gekennzeichnet, dass** die Übertragungseinrichtungen (24) aus einem mit der Abtriebswelle des Elektromotors (3) fest verbundenen ersten Zahnrad (25) bestehen, das in ein zweites Zahnrad (26) größerer Abmessungen als das erste eingreift, wobei das zweite Zahnrad (26) um eine Drehachse montiert ist, die im Inneren des Trägergehäuses (20) geführt wird, wobei das zweite Zahnrad (26) fest mit einer ersten Kurbelwelle (27) verbunden ist, die drehfrei mit dem ersten Ende einer Verbindungsstange (28) zusammenwirkt, deren anderes Ende ebenfalls drehfrei um eine zweite Kurbelwelle (29) montiert ist, die mit der zylindrischen Muffe (21a) der Bohrung (21) fest verbunden ist, um letztere in eine Schwingbewegung anzutreiben.

## Claims

1. Drilling device for forming a channel of curved profile inside the body of a vertebra of a spinal segment of a vertebral column to be treated, **characterized in that** it is composed of an articulated drill (1) provided with a metal tube (10) comprising, along its longitudinal axis XX', and at one of its ends, fixing means (11) that cooperate with a device (2) for driving it in an oscillatory movement, while the other end comprises, on the one hand, a profile (12) with a succession of alternately concave and convex loops (13) ensuring the deformation of said profile along a radius of curvature r, and, on the other hand, at least one series of teeth (15, 16) with cutting edges (17, 18).

2. Drilling device according to Claim 1, **characterized in that** the first series of teeth (15) has a cutting edge (17) which is inclined with respect to the longitudinal axis XX' of the metal tube (10) by an angle α of between 5 and 15 degrees.

3. Drilling device according to Claim 2, **characterized in that** the second series of teeth (16) has a cutting edge (18) which is inclined with respect to the longitudinal axis XX' of the metal tube (10) by an angle β of between 55 and 80 degrees.

4. Drilling device according to Claim 1, **characterized in that** the metal tube (10) has, in its inner part, a protective sheath (19) of flexible material defining an internal bore (19a) for internally smoothing the roughnesses and the discontinuities of the free end of the profile (12) formed by the succession of alternately concave and convex loops (13).

5. Drilling device according to Claim 1, **characterized in that** the profile (12) is formed by a combination of loops (13) constituted, on the periphery of the metal tube (10), by teeth (13a to 13e) cooperating respectively in seats (13f to 13j) in such a way as to constitute an asymmetrical and alternately concave and convex profile.

6. Drilling device according to Claim 5, **characterized in that** the profile (12) has, on the external fibre or face Fs of the radius of curvature r, the upper teeth (13a), adjacent teeth (13b), intermediate teeth (13c) and lateral teeth (13d), and, on the internal fibre or face Fi, the notched teeth (13e).

7. Drilling device according to Claim 5, **characterized in that** each loop (13) has, between the edges of the tooth (13a to 13e) and the edges of the corresponding seat (13f to 13j), a functional play of positive axial displacement Jp, a functional play of negative axial displacement Jn, and a functional play of angulation Ja allowing the profile (12) of the metal tube (10), on the one hand, to curve progressively without unlocking of the upper tooth (13a) and of the adjacent teeth (13b) until reaching the radius of curvature r, and, on the other hand, to withstand the stresses of the oscillatory movements.

8. Drilling device according to Claim 1, **characterized in that** the fixing means (11) are composed of a cylindrical sleeve (4) cooperating with a bore (21) of complementary shape formed in a cylindrical sleeve (21a) free in rotation and guided axially inside a support housing (20) of the drive device (2).

9. Drilling device according to Claim 8, **characterized in that** the cylindrical sleeve (4) has a flattened surface (5) provided at its centre with an angular indexing strip (6) cooperating with a groove (22) of the same profile formed in the bore (21) of the support housing (20).

10. Drilling device according to Claim 8, **characterized in that** the cylindrical sleeve (4) is provided, at one of its ends, with a peripheral abutment (7) for blocking the engagement in translation of the metal tube (10) inside the bore (21).

11. Drilling device according to Claim 8, **characterized in that** the support housing (20) of the drive device (2) has, below the bore (21) and counter to the direction of introduction of the metal tube (10), means (23) for receiving and fixing an electric motor (3).

12. Drilling device according to Claim 8, **characterized in that** the drive device (2) comprises, inside the support housing (20), transmission means (24) for converting the movement of continuous rotation of the electric motor (3) into an oscillatory or reciprocating movement with an amplitude of at least 60 degrees.

13. Drilling device according to Claim 8, **characterized in that** the transmission means (24) are composed of a first toothed wheel (25) integral with the output shaft of the electric motor (3) and meshing with a second toothed wheel (26) of greater dimensions than the first, said second toothed wheel (26) being mounted about a rotation axle which is guided inside the support housing (20), said second toothed wheel (26) being integral with a first crankshaft (27) cooperating freely in rotation with the first end of a connecting rod (28), of which the other end is mounted, also freely in rotation, about a second crankshaft (29) integral with the cylindrical sleeve (21a) of the bore (21) in order to drive the cylindrical sleeve (21a) in an oscillatory movement.
